(19) **Europäisches Patentamt**

**European Patent Office**

**Office européen des brevets**

(11) **EP 0 914 820 B1**

(12) **EUROPÄISCHE PATENTSCHRIFT**

(45) Veröffentlichungstag und Bekanntmachung des Hinweises auf die Patenterteilung:
**10.08.2005 Patentblatt 2005/32**

(51) Int Cl.⁷: **A61K 9/70**, A61L 15/58

(21) Anmeldenummer: **98119834.4**

(22) Anmeldetag: **20.10.1998**

(54) **Wirkstoffhaltige Pflaster mit partiell aufgebrachter Klebemasse**

Plasters containing active agents having a partially applied adhesive composition

Emplâtres contenant des médicaments pourvu d'une composition adhésive partiellement appliquée

(84) Benannte Vertragsstaaten:
**AT DE ES FR GB IT NL SE**

(30) Priorität: **08.11.1997 DE 19749467**

(43) Veröffentlichungstag der Anmeldung:
**12.05.1999 Patentblatt 1999/19**

(73) Patentinhaber: **Beiersdorf Aktiengesellschaft**
**20245 Hamburg (DE)**

(72) Erfinder:
• **Himmelsbach, Peter**
**21614 Buxtehude (DE)**
• **Jauchen, Peter**
**22455 Hamburg (DE)**
• **Broschk, Katharina**
**20253 Hamburg (DE)**
• **Köhler, Ulrich Dr.**
**22089 Hamburg (DE)**
• **Wasner, Matthias**
**21029 Hamburg (DE)**

(56) Entgegenhaltungen:
WO-A-97/43992          WO-A-97/43993
DE-A- 4 242 015        US-A- 5 065 752
US-A- 5 389 168

• DATABASE WPI Section Ch, Week 8240 Derwent Publications Ltd., London, GB; Class A96, AN 82-84631E XP002092226 & JP 57 139347 A (SEKISUI CHEM IND CO LTD) , 28. August 1982

**Beschreibung**

Die Erfindung betrifft wirkstoffhaltige Pflaster mit einem Trägermaterial und einer darauf zumindest partiell aufgebrachten Klebemasse, welche den Wirkstoff oder gegebenenfalls auch mehrere Wirkstoffe enthält, die an die Haut abgegeben werden.

Transdermale Therapeutische Systeme (TTS) sind Darreichungsformen von Medikamenten, die einen oder mehrere Arzneistoffe über einen definierten Zeitraum an ihrem Anwendungsort an die Haut abgeben. Es wird dabei zwischen systemisch und lokal wirksamen Darreichungsformen unterschieden.
Bei systemisch wirkenden Darreichungsformen gelangt der Wirkstoff durch Diffusion durch die Haut in den Blutkreislauf und kann im ganzen Körper wirken. Lokal wirkende Darreichungsformen wirken dagegen nur an den applizierten Stellen. Der Wirkstoff verbleibt in der Haut beziehungsweise in den darunter liegenden Schichten.

Stark klebende Pflaster werden üblicherweise vollflächig mit einer Zink-Kautschuk-Klebemasse beschichtet. Das Verkleben derartiger Produkte auf der Haut zeigt nach dem Ablösen deutliche Hautirritationen und eine mechanische Beanspruchung der Haut. Die Verklebung läßt sich ohne Hilfsmittel nur unter Schmerzen lösen. Fallweise kommt es zu allergischen Reaktionen. Die verwendeten Klebemassen führen darüber hinaus oft zu einem Massetransfer, d. h., einem Umspulen, auf die Haut.

Die Verwendung von hautfreundlichen Klebemassen, wie Acrylatklebemassen und Hydrogele, ist aufgrund der geringen Scherstabilität und Anfaßklebrigkeit nicht erwägenswert. Eine Verbesserung durch eine Nachbehandlung, insbesondere Vernetzung, ist möglich, jedoch bleibt das Ergebnis insgesamt unbefriedigend. Die propriorezeptive Wirkung ist gegenüber den Systemen mit einer Zink-Kautschuk-Klebemasse geringer.

Andere bekannte Klebesysteme auf Basis von herkömmlichen Blockcopolymeren sind zum einen durch hohen Stabilisatorzusatz nicht hautfreundlich beziehungsweise zeigen durch die hohe Kohäsivität bislang nur eine Eignung für technische Anwendungsfälle, zum anderen sind sie nicht stark hautklebend und hauthaftend einzustellen.

Die zuvor genannten Klebemassen sind druckempfindliche Selbstklebemassen, wobei die Massen für die Verarbeitung in einer Trägermatrix vorliegen können. Als Trägermatrix werden gängige organische oder anorganische Lösemittel oder Dispergiermittel verstanden.

Systeme ohne Trägermatrix werden als 100 %-Systeme bezeichnet und sind ebenfalls nicht unbekannt. Sie werden im elastischen oder thermoplastischen Zustand verarbeitet. Eine gängige Verarbeitungsweise ist die Schmelze. Auch solche Haftschmelzklebemassen sind im Stande der Technik bereits vorbeschrieben. Sie basieren auf natürlichen oder synthetischen Kautschuken und/oder anderen synthetischen Polymeren.

Vorteilhaft an den 100 %-Systemen ist, dass bei ihnen verfahrenstechnisch ein Entfernen der Trägermatrix, d.h. der Hilfsmittel vermieden wird, wodurch sich die Verarbeitungsproduktivität steigert und sich gleichzeitig der Maschinen- und Energieaufwand reduziert. Weiter wird so ein Verbleiben von Reststoffen der Trägermatrix reduziert. Dieses begünstigt wiederum die Senkung des allergenen Potentials.

Aufgrund ihrer hohen Härte ist für solche 100 %-Systeme die Hauthaftung problematisch.

Es ist ferner bekannt, derartige Selbstklebemassen nicht nur vollflächig sondern auch rasterpunktförmig aufzubringen, beispielsweise durch Siebdruck (DE-PS 42 37 252), wobei die Klebstoffpünktchen auch unterschiedlich groß und/oder unterschiedlich verteilt sein können (EP-PS 353 972), oder durch Tiefdruck von in Längs- und Querrichtung zusammenhängenden Stegen (DE-PS 43 08 649).

Der Vorteil des rasterförmigen Auftrags besteht darin, dass die Klebematerialien bei entsprechend porösem Trägermaterial luft- und wasserdampfdurchlässig sowie in der Regel leicht wieder ablösbar sind.

Ein Nachteil dieser Produkte besteht jedoch in der Tatsache, dass bei zu hoher Flächendeckung der an sich undurchlässigen Klebeschicht die Luft- und Wasserdampfdurchlässigkeit sich entsprechend verringert, sowie der Klebemassenverbrauch steigt und bei geringer Flächendeckung der Klebeschicht die Klebeeigenschaften leiden, d.h., das Produkt löst sich, insbesondere bei schweren, textilen Trägermaterialien, zu leicht vom Untergrund.

Im Stande der Technik sind bereits zahlreiche Ausführungsformen wirkstoffhaltiger Pflaster beschrieben worden, die zum Teil nach dem Reservoirprinzip arbeiten, bei welchem der Wirkstoff beispielsweise über eine Membran abgegeben wird, teilweise auch mit einem Matrixsystem oder mit komplizierterem mehrschichtigen Aufbau.

Dann ist bekannt, dass die Klebemasse des Pflasters als den Wirkstoff enthaltende Matrix eingesetzt werden kann. Neben aus Lösung aufgetragenen Selbstklebemassen werden auch Heißschmelzklebemassen dafür vorgeschlagen, zum Beispiel in der EP-A 663 431, EP-A 452 034, EP-A 305 757, DE-OS 43 10 012, DE-OS 42 22 334 und DE-C 42 24 325. Als Wirkstoffe werden hierbei, wenn diese benannt werden, systemisch wirkende aufgeführt.

Beispielhaft für wirkstoffhaltige Pflaster seien die durchblutungsfördernden Wirkstoffpflaster genannt, die zu der Gruppe der lokal wirksamen therapeutischen Systeme gehören. Die Anwendung solcher Pflaster ist angezeigt zur Behandlung von rheumatischen Beschwerden, Ischias, Hexenschuss, Nackensteifigkeit, Schulter-Arm-Schmerzen sowie Muskelverspannungen und -zerrungen, Muskelkater oder Muskel-, Gelenk- und Nervenschmerzen im Bereich des Bewegungsapparates.

Capsaicin, Belladonna und Nonivamid sind bekannte Wirkstoffe solcher lokal, durchblutungsfördernd wirkender Pflaster. Aufgrund ihrer Anwendung am Bewegungsapparat müssen sie in der Regel stark kleben. Üblicherweise werden die Pflaster vollflächig mit einer Harz-Kautschuk-Klebemasse beschichtet, welche den Wirkstoff enthält.

Derartige Pflaster, welche meist größer flächig aufgebracht werden müssen, zeigen jedoch nach dem Ablösen bei empfindlichen Patienten z. Teil deutliche mechanische Hautirritationen. Fallweise kommt es zu allergischen Reaktionen. Ihr Entfernen ist nach längerer Tragezeit bis zu einem gewissen Grade schmerzhaft.

Ein weiterer Nachteil der bekannten wärmewirksamen Pflaster mit einer Klebemasse auf Basis von natürlichem Kautschuk, die in Form einer Lösung mit organischen Lösungsmitteln auf den Pflasterträger aufgetragen wird, ist die vergleichsweise niedrige Freisetzungsrate des Wirkstoffs.

Die geschilderten und weitere Nachteile treffen ebenso auch auf wirkstoffhaltige Pflaster zu, die andere als die genannten Stoffe beinhalten.

So beschreibt die WO 94/02123 ein Wirkstoffpflaster auf Basis von Haftschmelzklebemassen, welche niedrigschmelzende und/oder leicht flüchtige Wirkstoffe in einer Konzentration von 2,5 Gew.-% bis 25 Gew.-% enthält. Bei den dort eingesetzten Polymeren handelt es sich um A-B-A-Dreiblock Styrol-Ethylen-Butylen-Styrol Blockcopolymerisate, welche sich durch einen geringen Initialtack und geringe Klebkraft auf Haut auszeichnen.

EP 0663431 A2, EP 0443759 A3, EP 0452034 A2 und US 5371128 beschreiben Verwendungen von druckempfindlichen Schmelzhaftklebern auf Silikonbasis mit diversen Additiven und in differenzierten Aufbauformen.

DE 43 10 012 A1 beschreibt den Aufbau eines dermalen therapeutischen Systems aus schmelzfähigen Poly(meth)acrylat-Mischungen.

DE 43 16 751 C1 beschreibt ein Mehrkammersystem zur Darreichung von Wirkstoffen.

EP 0439180 beschreibt ein Wirkstoffpflaster zur Darreichung von Tulobuterol.

EP 0305757 beschreibt ein Wirkstoffpflaster zur Darreichung von Nicotin.

EP 0305758 beschreibt ein Wirkstoffpflaster zur Darreichung von Nitroglycerin.

EP 0305756 beschreibt eine Vorrichtung von Stoffen und ihrer Herstellung und Verwendung.

DE 37 43 945 A1 beschreibt eine Vorrichtung zur Abgabe von Stoffen sowie das Verfahren zur Herstellung dieser Vorrichtung. Bei der beschriebenen Haftschmelzklebemasse auf Basis von SIS handelt es sich um keine selbstklebende Vorrichtung. Die dort angegebenen Verarbeitungsbereiche liegen deutlich unter denen von Heißschmelzklebemassen und würden für solche beschriebenen Systeme keine ausreichende Verankerung der Klebemasse bieten.

WO 96/22083 zeigt einen Polyisobutylenkleber für transdermale Zwecke, welcher einen Klebrigmacher mit hohem Glasübergangspunkt hat. Der Kleber ist nicht geschäumt.

JP 07-196505 beschreibt eine Darreichung von Inkomethacin in Schmelzhaftklebern. Es wird hier ein Polyethylenschaum als Trägermaterial verwendet.

JP 59-155479 beschreibt eine Klebemasse, welche auf Basis eines Elastomers durch Reaktion einen vernetzten Schaum ergibt. Eine Freisetzung von Stoffen ist nicht beschrieben.

JP 08-092954 offenbart einen Wärmeumschlag, welcher ein Lokalanästetikum enthält. Die verwendete Klebemasse ist keine haftklebrige oder selbstklebende Masse, sondern ein Laminatkleber.

US 5389168 offenbart einen klebenden Schaum; dieser wird jedoch nur ausschließlich als Laminatkleber verwendet. Eine Freisetzung von Stoffen ist nicht beschrieben.

JP 08-175979 offenbart ein wirkstoffhaltiges Pflaster mit einem Trägermaterial und einer darauf partiell aufgebrachten Heißschmelzklebemasse. Die Heißschmelzklebemasse kann ein A-B-A- Blockcopolymer sein und einen oder mehrere Wirkstoffe enthalten. Die Klebemasse zudem kann geschäumt und offenporig sein. Allerdings bezieht sich die angegebene Luftdurchlässigkeit lediglich auf eine ein wärmenden Körper enthaltenden Folie. Diese Luftdurchlässigkeit ist zwangsläufig erforderlich, da der wärmende Körper Eisenverbindungen aufweist. Dieser luftdurchlässige Film dient einzig und allein als Bestandteil eines Beutels in dem das wärmeabstrahlende Element enthalten ist und beschreibt nicht das Pflaster als solches. Die Luftdurchlässigkeit dieses den Beutel bildenden Films ist für das wärmeabstrahlende Element zwingend notwendig, da wärmeabstrahlende Elemente benutzt werden, die unter Lufteinfluss sich Erwärmen.

Aufgabe der Erfindung war es deshalb, wirkstoffhaltige Pflaster zur Verfügung zu stellen, welche sich bei Vermeidung der aus dem Stand der Technik bekannten Nachteile durch eine gute Wirksamkeit, d.h., eine relativ hohe Freisetzungsrate, und gute Hautverträglichkeit bei gleichzeitig gutem Klebeverhalten auszeichnen. Auch sollen sie sich technisch wenig aufwendig und umweltverträglich herstellen lassen.

Gelöst wird diese Aufgabe durch wirkstoffhaltige Pflaster gemäß Anspruch 1. Gegenstand der Unteransprüche sind vorteilhafte Ausführungsformen der erfindungsgemäßen Pflaster. Des weiteren umfasst die Erfindung aber auch Verfahren zur Herstellung derartiger Pflaster.

Demgemäß betrifft die Erfindung wirkstoffhaltige Pflaster mit einem Trägermaterial und einer darauf partiell aufgebrachten Heißschmelzklebemasse, die sich dadurch auszeichnen, dass die Heißschmelzklebemasse

    a) 10 Gew.% bis 90 Gew.% A-B/A-B-A- Blockcopolymeren mit einem Anteil an Di-Blockcopolymeren von weniger als 80 Gew.% enthält,

b) wobei Phase A vornehmlich Polystyrol oder dessen Derivate sind und der Gesamtstyrolanteil in der Klebemasse niedriger als 35 Gew.% beträgt,

c) 5 Gew.% bis 80 Gew.% Klebrigmacher, insbesondere Öle, Wachse, Harze und/oder deren Mischungen,

d) weniger als 60 Gew.% Weichmacher,

e) weniger als 15 Gew.% Additive,

f) weniger als 5 Gew.% Stabilisatoren und

g) 0,01 Gew.% bis 10 Gew.% Wirkstoff oder Wirkstoffe enthält und

h) eine dynamisch-komplexe Glasübergangstemperatur bei einer Frequenz von 0,1 rad/s von weniger als 10 °C aufweist,

i) die offenporige Klebemasse mittels dem Schaum-Mix-System mit einem Schäumungsgrad von mindestens 5 Vol.-% geschäumt ist und

j) in Form von polygeometrischen Kalotten auf das Trägermaterial aufgebracht ist und

dass das mit Klebemasse beschichtete Trägermaterial eine Luftdurchlässigkeit größer 1 $cm^3/cm^2 * s)$ und eine Wasserdampfdurchlässigkeit größer 500 $g/(m^2*24h)$ aufweist.

Unter der Bezeichnung "Wirkstoffe" im Zusammenhang mit der vorliegenden Erfindung werden chemische Elemente, organische und anorganische Verbindungen verstanden, die aus den sie enthaltenden Bestandteilen eines gattungsgemäßen Pflasters herauswandern können und dadurch einen angestrebten Effekt hervorrufen. Unter den Anwendungsgebieten der erfindungsgemäßen Pflaster ist die Human- und Tiermedizin von besonderer Bedeutung. Typische, über erfindungsgemäß hergestellte Pflaster verabreichbare Stoffe sind hierbei:

Aceclidin, Amfetaminil, Amfetamin, Amylnitrit, Apophedrin, Atebrin, Alpostadil, Azulen, Arecolin, Anethol, Amylenhydrat, Acetylcholin, Acridin, Adenosintriphosphorsäure, L-Äpfelsäure, Alimemazin, Allithiamin, Allyl Isothiocyanat, Aminoethanol, Apyzin, Apiol, Azatadin, Alprenolol, Äthinazon, Benzoylperoxid, Benzylalkohol, Bisabolol, Bisnorephedrin, Butacetoluid, Benactyzin, Campher, Colecalciferol, Chloralhydrat, Clemastin, Chlorobutanol, Capsaicin, Cyclopentamin, Clobutinol, Chamazulen, Dimethocain, Codein, Chloropromazin, Chinin, Chlorthymol, Cyclophosphamid, Cinchocain, Chlorambuzil, Chlorphenesin, Diethylethan, Divinylethan, Dexchlorpheniramin, Dinoproston, Dixyrazin, Ephedrin, Ethosuximid, Enallylpropymal, Emylcamat, Erytroltetranitrat, Emetin, Enfluran, Eucalyptol, Etofenamat, Ethylmorphin, Fentanyl, Fluanison, Guajazulen, Halothan, Hyoscyamin, Histamin, Fencarbamid, Hydroxycain, Hexylresorcin, Isoaminilcitrat, Isosorbiddinitrat, Ibuprofen, Jod, Jodoform, Isoaminil, Lidocain, Lopirin, Levamisol, Methadon, Methyprylon, Methylphenidat, Mephenesin, Methylephedrin, Meclastin, Methopromazin, Mesuximid, Nicethamid, Norpseudoephedrin, Menthol, Methoxyfluran, Methylpentinol, Metixen, Mesoprostol, Oxytetracain, Oxyprenolol, Oxyphenbutazon, Oxychinolin, Pinen, Prolintan, Procyclidin, Piperazin, Pivazid, Phensuximid, Procain, Phenindamin, Promethazin, Pentetrazol, Profenamin, Perazin, Phenol, Pethidin, Pilocarpin, Prenylamin, Phenoxybenzamin, Resochin, Scopolamin, Salicylsäureester, Spartein, Trichlorethylen, Timolol, Trifluperazin, Tetracain, Trimipramin, Tranylcypromin, Trimethadion, Tybamat, Thymol, Thioridazin, Valproinsäure, Verapamil, sowie weitere, dem Fachmann geläufige, über die Haut, eingeschlossen Schleimhäute, aufnehmbare Wirkstoffe. Selbstverständlich ist diese Aufzählung nicht abschließend.

Die Verteilung der Wirkstoffe in der Klebemasse erfolgt vorzugsweise in einem Thermohomogenisator wie zum Beispiel Thermomixer, Thermokneter, Walzenwerke oder Schneckensysteme. Die Zugabe des Wirkstoffs kann in die vollständig hergestellte Klebemasse erfolgen. Beispielhaft kann der Wirkstoff auch in eine Zwischenstufe oder in die Ausgangsmischung eingearbeitet werden.

Als Klebemassen lassen sich vorteilhafterweise thermoplastische Heißschmelzklebemassen einsetzen auf Basis natürlicher und synthetischer Kautschuke und anderer synthetischer Polymere wie Acrylate, Methacrylate, Polyurethane, Polyolefine, Polyvinylderivate, Polyester oder Silikone mit entsprechenden Zusatzstoffen wie Klebharzen, Weichmachern, Stabilisatoren und anderen Hilfsstoffen soweit erforderlich.

Ihr Erweichungspunkt sollte höher als 50°C liegen, da die Applikationstemperatur in der Regel mindestens 90°C beträgt, bevorzugt zwischen 120°C und 150 °C beziehungsweise 180°C und 240 °C bei Silikonen. Gegebenenfalls kann eine Nachvernetzung durch UV- oder Elektronenstrahlen-Bestrahlung angebracht sein.

Heißschmelzklebemassen auf Basis von Blockcopolymeren zeichnen sich durch ihre vielfältige Variationsmöglichkeiten aus, denn durch die gezielte Absenkung der Glasübergangstemperatur der Selbstklebemasse infolge der Auswahl der Klebrigmacher, der Weichmacher sowie der Polymermolekülgröße und der Molekularverteilung der Einsatzkomponenten wird die notwendige funktionsgerechte Verklebung mit der Haut auch an kritischen Stellen des menschlichen Bewegungsapparates gewährleistet.

Die hohe Scherfestigkeit der Heißschmelzklebemasse wird durch die hohe Kohäsivität des Polymeren erreicht. Die gute Anfassklebrigkeit ergibt sich durch die eingesetzte Palette an Klebrigmachern und Weichmachern.

Die Heißschmelzklebemasse basiert auf A-B-, A-B-A-Blockcopolymere oder deren Mischungen. Die harte Phase A ist vornehmlich Polystyrol oder dessen Derivate, und die weiche Phase B enthält Ethylen, Propylen, Butylen, Butadien, Isopren oder deren Mischungen, hierbei besonders bevorzugt Ethylen und Butylen oder deren Mischungen.

Polystyrolblöcke können aber auch in der weichen Phase B enthalten sein, und zwar bis zu 20 Gew.-%. Der gesamte Styrolanteil sollte aber stets niedriger als 35 Gew.-% liegen. Bevorzugt werden Styrolanteile zwischen 5% und 30%, da ein niedrigerer Styrolanteil die Klebemasse anschmiegsamer macht.

Die gezielte Abmischung von Di-Block- und Tri-Blockcopolymeren ist vorteilhaft, wobei ein Anteil an Di-Blockcopolymeren von kleiner 80 Gew.-% erhalten wird.

Die Heißschmelzklebemasse weist die nachfolgend angegebene Zusammensetzung auf:

| | |
|---|---|
| 10 Gew.-% bis 90 Gew.-% | Blockcopolymere, |
| 5 Gew.-% bis 80 Gew.-% | Klebrigmacher wie Öle, Wachse, Harze und/oder deren Mischungen, bevorzugt Mischungen aus Harzen und Ölen, |
| weniger als 60 Gew.-% | Weichmacher, |
| weniger als 15 Gew.-% | Additive, |
| weniger als 5 Gew.-% | Stabilisatoren, |
| 0,01 Gew.-% bis 10 Gew.-% | Wirkstoff oder Wirkstoffe. |

Die als Klebrigmacher dienenden aliphatischen oder aromatischen Öle, Wachse und Harze sind bevorzugt Kohlenwasserstofföle, -wachse und -harze, wobei sich die Öle, wie Paraffinkohlenwasserstofföle, oder die Wachse, wie Paraffinkohlenwasserstoffwachse, durch ihre Konsistenz günstig auf die Hautverklebung auswirken. Als Weichmacher finden mittel- oder langkettige Fettsäuren und/oder deren Ester Verwendung. Diese Zusätze dienen dabei der Einstellung der Klebeeigenschaften und der Stabilität. Gegebenenfalls kommen weitere Stabilisatoren und andere Hilfsstoffe zum Einsatz.

Ein Füllen der Klebemasse mit mineralischen Füllstoffen, Fasern, Mikrohohl- oder - vollkugeln ist möglich.

Die Heißschmelzklebemasse weist einen Erweichungspunkt auf oberhalb von 50 °C, bevorzugt 70 °C bis 220 °C, ganz besonders bevorzugt 75 °C bis 140 °C.

Die Heißschmelzklebemassen sind vorzugsweise so eingestellt, daß sie bei einer Frequenz von 0,1 rad/s eine dynamisch-komplexe Glasübergangstemperatur von weniger als 10 °C, bevorzugt von 0 °C bis -30 °C, ganz besonders bevorzugt von -6 °C bis -25 °C, aufweisen.

Insbesondere an Pflaster werden hohe Anforderungen bezüglich der Klebeeigenschaften gestellt. Für eine ideale Anwendung sollte die Heißschmelzklebemasse eine hohe Anfassklebrigkeit besitzen. Die funktionsangepasste Klebkraft auf der Haut und auf der Trägerrückseite sollte vorhanden sein. Weiterhin ist, damit es zu keinem Verrutschen kommt, eine hohe Scherfestigkeit der Heißschmelzklebemasse notwendig.

Durch die gezielte Absenkung der Glasübergangstemperatur der Selbstklebemasse infolge der Auswahl der Klebrigmacher, der Weichmacher sowie der Polymermolekülgröße und der Molekularverteilung der Einsatzkomponenten wird die notwendige funktionsgerechte Verklebung mit der Haut und der Trägerrückseite erreicht.

Die hohe Scherfestigkeit der hier eingesetzten Selbstklebemasse wird durch die hohe Kohäsivität des Blockcopolymeren erreicht. Die gute Anfassklebrigkeit ergibt sich durch die eingesetzte Palette an Klebrigmachern und Weichmachern.

Die Produkteigenschaften wie Anfassklebrigkeit, Glasübergangstemperatur und Scherstabilität lassen sich mit Hilfe einer dynamisch-mechanischen Frequenzmessung gut quantifizieren. Hierbei wird ein schubspannungsgesteuertes Rheometer verwendet.

Die Ergebnisse dieser Meßmethode geben Auskunft über die physikalischen Eigenschaften eines Stoffes durch die Berücksichtigung des viskoelastischen Anteils. Hierbei wird bei einer vorgegebenen Temperatur die Heißschmelzklebemasse zwischen zwei planparallelen Platten mit variablen Frequenzen und geringer Verformung (linear viskoelastischer Bereich) in Schwingungen versetzt. Über eine Aufnahmesteuerung wird computerunterstützt der Quotient ($Q = \tan \delta$) zwischen dem Verlustmodul (G" viskoser Anteil) und dem Speichermodul (G' elastischer Anteil) ermittelt.

$$Q = \tan \delta = G''/G'$$

Für das subjektive Empfinden der Anfassklebrigkeit (Tack) wird eine hohe Frequenz gewählt sowie für die Scherfestigkeit eine niedrige Frequenz.

Eine hoher Zahlenwert bedeutet eine bessere Anfaßklebrigkeit und eine schlechtere Scherstabilität.

Die Glasübergangstemperatur ist die Temperatur, bei der amorphe oder teilkristalline Polymere vom flüssigen oder gummielastischen Zustand in den hartelastischen oder glasigen Zustand übergehen oder umgekehrt (Römpp Chemie-Lexikon, 9. Aufl., Band 2, Seite 1587, Georg Thieme Verlag Stuttgart - New York, 1990). Er entspricht dem Maximum der Temperaturfunktion bei vorgegebener Frequenz. Besonders für medizinische Anwendungen ist ein relativ niedriger Glasübergangspunkt notwendig.

| Bezeichnung | $T_G$ niedrige Frequenz | Anschmiegsamkeit niedrige Frequenz/RT | Anfaßklebrigkeit hohe Frequenz/RT |
|---|---|---|---|
| Heißschmelzselbstklebemasse A | -12 ± 2°C | tan δ = 0,88 ± 0,03 | tan δ = 0,84 ± 0,03 |
| Heißschmelzselbstklebemasse B | -9 ± 2°C | tan δ = 0,32 ± 0,03 | tan δ = 1,70 ± 0,03 |

Bevorzugt werden erfindungsgemäß Heißschmelzklebemassen, bei denen das Verhältnis des viskosen Anteils zum elastischen Anteil bei einer Frequenz von 100 rad/s bei 25 °C größer 0,7 ist, bevorzugt 1,0 bis 5,0, oder Heißschmelzklebemassen, bei denen das Verhältnis des viskosen Anteils zum elastischen Anteil bei einer Frequenz von 0,1 rad/s bei 25°C kleiner 0,6 ist, bevorzugt zwischen 0,4 und 0,02, ganz besonders bevorzugt zwischen 0,3 und 0,1.

Um die funktionsgerechte Verwendung der Pflaster sicherzustellen, sind die Klebemassen geschäumt.

Die mit den Wirkstoffen versehenen Klebemassen werden dabei bevorzugt mit inerten Gasen wie Stickstoff, Kohlendioxid, Edelgasen, Kohlenwasserstoffen oder Luft oder deren Gemischen geschäumt. In manchen Fällen hat sich ein Aufschäumen zusätzlich durch thermische Zersetzung gasentwickelnder Substanzen wie Azo-, Carbonat- und Hydrazid-Verbindungen als geeignet erwiesen.

Der Schäumungsgrad, d.h. der Gasanteil, beträgt mindestens etwa 5 Vol.-% und kann bis zu etwa 85 Vol.-% reichen. In der Praxis haben sich Werte von 10 Vol.-% bis 75 Vol.-%, bevorzugt 50 Vol.-%, gut bewährt. Wird bei relativ hohen Temperaturen von ungefähr 100 °C und vergleichsweise hohem Innendruck gearbeitet, entstehen sehr offenporige Klebstoffschaumschichten, die besonders gut luft- und wasserdampfdurchlässig sind.

Die vorteilhaften Eigenschaften der erfindungsgemäßen Klebebeschichtungen, wie geringer Klebstoffverbrauch, hohe Anfassklebrigkeit und gute Anschmiegsamkeit auch an unebenen Flächen durch die Elastizität und Plastizität der geschäumten Klebemassen sowie der Initialtack lassen sich auf dem Gebiet der Wirkstoffpflaster optimal nutzen.

Gleichzeitig wird durch die Vakuolen im Schaum der Transport der Wirkstoffe überproportional gesteigert, womit sehr gute Freisetzungsraten erreicht werden.

Das Verfahren zur Herstellung der erfindungsgemäß geschäumten Klebemasse arbeitet nach dem Schaum-Mix-System. Hierbei wird die thermoplastische Klebemasse unter hohem Druck bei einer Temperatur über dem Erweichungspunkt (ungefähr 120°C) mit den vorgesehenen Gasen wie zum Beispiel Stickstoff, Luft oder Kohlendioxid in unterschiedlichen Volumenanteilen (etwa 10 Vol.-% bis 80 Vol.-%) in einem Stator/Rotorsystem umgesetzt. Während der Gasvordruck größer 100 bar ist, betragen die Mischdrucke Gas/Thermoplast im System 40 bis 100 bar, bevorzugt 40 bis 70 bar. Der so hergestellte Haftklebeschaum kann anschließend über eine Leitung in das Auftragswerk gelangen. Bei dem Auftragswerk finden handelsübliche Düsen, Extruder- oder Kammersysteme Verwendung.

Die Wechselwirkung der Wirkstoffe mit der Haut wird bekanntlich mit in die Klebemasse eingemischten Enhancern moduliert beziehungsweise durch den oklusiven Effekt von Klebemasse und Abdeckung verstärkt. Im Gegensatz hierzu kann durch den Einsatz von atmungsaktiven dotierten Beschichtungen in Verbindung mit elastischen ebenfalls atmungsaktiven Trägermaterialien insbesondere zum Beispiel während sportlicher Betätigungen

a) ein vom Anwender subjektiv angenehmer empfundener Tragekomfort und

b) eine vom Freisetzungsverhalten durch die mit der Umgebung ungestörtere Wechselwirkung (zum Beispiel Unterdrückung von Körperschweiß) der Haut eine definierter Penetration von Wirkstoffen in die Haut erzielt werden.

Umgekehrt lässt sich durch die hier genannten Verfahren auch eine Durchlässigkeit des dotierten Pflastersystems von außen erzielen. Mit dieser Produkteigenschaft können somit von außen durch den Träger Substanzen an die Kontaktstelle dotierter Kleber/Haut auch zu späteren Zeiten nach der eigentlichen Applikation herangebracht werden (Flüssigkeit aufträufeln, wischen, etc.). Diese Substanzen könnten zum Beispiel eine zusätzliche Enhancerwirkung beinhalten oder die medikamentöse Wirkung starten, schwächen oder für eine entsprechende Konsumentenauslobung geeignet modulieren.

Zur Verdeutlichung der Vorteile einer geschäumten Heißschmelzklebemasse gegenüber einer nicht-geschäumten sind ebenfalls mehrere Versuchsreihen in dem schubspannungsgesteuerten Rheometer durchgeführt worden. Es wurden verschiedene Heißschmelzklebemassen gewählt.

Dabei wurden wiederum bei einer vorgegebenen Temperatur geschäumte und nicht geschäumte Heißschmelzklebemassen zwischen zwei planparallelen Platten mit variablen Frequenzen und geringer Verformung (linear viskoelastischer Bereich) in Schwingungen versetzt. Anschließend wurde computerunterstützt der Quotient (Q = tan δ) zwischen dem Verlustmodul (G" viskoser Anteil) und dem Speichermodul (G' elastischer Anteil) ermittelt.

$$Q = \tan \delta = G''/G'$$

In der anschließenden Tabelle wird der Einfachheit halber Heißschmelzklebemasse mit HSKB abgekürzt.

HSKB A          Acrylat-Basis
HSKB B und C     Blockcopolymeren-Basis

| Bezeichnung | Anschmiegsamkeit niedrige Frequenz/RT | Anfaßklebrigkeit hohe Frequenz/RT |
|---|---|---|
| HSKB A (nicht geschäumt)<br>HSKB A Schaum Vol. ($N_2$)=50% | $\tan \delta = 0,35 \pm 0,05$<br>$\tan \delta = 0,46 \pm 0,05$ | $\tan \delta = 0,45 \pm 0,05$<br>$\tan \delta = 0,65 \pm 0,05$ |
| HSKB A (nicht geschäumt)<br>HSKB A Schaum Vol. ($N_2$) = 70% | $\tan \delta = 0,45 \pm 0,05$<br>$\tan \delta = 0,58 \pm 0,05$ | $\tan \delta = 0,50 \pm 0,05$<br>$\tan \delta = 0,68 \pm 0,05$ |
| HSKB B (nicht geschäumt)<br>HSKB B Schaum Vol. ($N_2$)=50% | $\tan \delta = 0,15 \pm 0,05$<br>$\tan \delta = 0,27 \pm 0,05$ | $\tan \delta = 1,7 \pm 0,05$<br>$\tan \delta = 1,85 \pm 0,05$ |
| HSKB C (nicht geschäumt)<br>HSKB C Schaum Vol.($N_2$)=50% | $\tan \delta = 0,16 \pm 0,03$<br>$\tan \delta = 0,31 \pm 0,05$ | $\tan \delta = 1,1 \pm 0,05$<br>$\tan \delta = 1,25 \pm 0,05$ |

Die Ergebnisse zeigen einen deutlichen Anstieg der tan δ-Werte durch die Schäumung, also eine messbar bessere Anschmiegsamkeit und Anfassklebrigkeit.
Durch die Schäumung der Klebemasse und die dadurch entstandenen offenen Poren in der Masse sind bei Verwendung eines an sich porösen Trägers die mit der Klebemasse beschichteten Produkte gut wasserdampf- und luftdurchlässig. Die benötigte Klebemassenmenge wird erheblich reduziert ohne Beeinträchtigung der Klebeeigenschaften. Die Klebemassen weisen eine überraschend hohe Anfassklebrigkeit (tack) auf, da pro Gramm Masse mehr Volumen und damit Klebeoberfläche zum Benetzen des zu beklebenden Untergrundes zur Verfügung steht und die Plastizität der Klebemassen durch die Schaumstruktur erhöht ist. Auch die Verankerung auf dem Trägermaterial wird dadurch verbessert. Außerdem verleiht die geschäumte Klebebeschichtung den Produkten ein weiches und anschmiegsames Anfühlen.
Durch das Schäumen wird zudem die Viskosität der Klebemassen in der Regel gesenkt. Hierdurch wird die Schmelzenergie erniedrigt und es können auch thermoinstabile Trägermaterialien direkt beschichtet werden.
Die Produktvorteile durch das Schäumen der Klebemassen wie hohe Klebkraft sowie gute Luft- und Wasserdampfdurchlässigkeit können aus den folgenden Messungen entnommen werden. In den Messungen wurden Pflasterbinden mit geschäumten beziehungsweise nicht-geschäumten Heißschmelzklebemassen beschichtet und miteinander verglichen.

**Beispiel 1**

Pflasterbinden mit geschäumten Heißschmelzklebemassen kleben stärker auf der Haut als Pflasterbinden mit ungeschäumten Heißschmelzklebemassen bei gleichem Masseauftrag. Da die Klebkraft auf der Haut von Hauttyp zu Hauttyp unterschiedlich ist, wurde die ungeschäumte Klebemasse mit einem Index 100 benannt und die geschäumte Pflasterbinde in Relation gebracht. Dieses ergab folgende Ergebnisse:

Index = Klebkraft auf der Haut (geschäumt)/Klebkraft auf der Haut (ungeschäumt)

Die anschließende Tabelle zeigt die Klebkraftsteigerung auf Haut.

| Masseauftrag Gewebe (unelast.) | Gewebe (elast.) | Vlies |
|---|---|---|
| 40 g/m$^2$ | 105-110% | |
| 60 g/m$^2$ | | 120-130% |

(fortgesetzt)

| Masseauftrag Gewebe (unelast.) | Gewebe (elast.) | Vlies |
|---|---|---|
| 80 g/m$^2$ | 110-125% | 110-120% |
| 120 g/m$^2$ | 120-170% | |

**Beispiel 2**

Pflasterbinden mit geschäumten Heißschmelzklebemassen sind luftdurchlässiger als Pflasterbinden mit ungeschäumten Heißschmelzklebemassen bei gleichem Masseauftrag. Die Luftdurchlässigkeit von Pflasterbinden mit nicht geschäumten Klebemassen betrug bei den überprüften Mustern weniger als 1 cm$^3$/cm$^2$/sec. Die Luftdurchlässigkeiten beziehen sich auf einen Schäumungsgrad von 50% im Endprodukt.

Die anschließende Tabelle zeigt die Luftdurchlässigkeit in Abhängigkeit vom Masseauftrag (jeweils in cm$^3$/cm$^2$/sec).

| Masseauftrag | Gewebe (unelast.) | Gewebe (elast.) ungedehnt | Gewebe (elast.) gedehnt | Vlies |
|---|---|---|---|---|
| 40 g/m$^2$ | 6-19 | | | |
| 60 g/m$^2$ | | | | 90-100 |
| 80 g/m$^2$ | 3 - 8 | 20 - 35 | 90 - 110 | |
| 120 g/m$^2$ | 0,5 - 3 | | | |

**Beispiel 3**

Die Luftdurchlässigkeit ist abhängig vom Schäumungsgrad. Die Ergebnisse wurden bei einem Masseauftrag von 80 g/m$^2$ ermittelt.

Die anschließende Tabelle zeigt die Abhängigkeit der Luftdurchlässigkeit vom Schäumungsgrad.

| Schäumungsgrad | unelast. Gewebe |
|---|---|
| 30% | 2 - 5 cm$^3$/cm$^2$/sec |
| 50% | 3 - 8 cm$^3$/cm$^2$/sec |
| 70% | 7 - 25 cm$^3$/cm$^2$/sec |

**Beispiel 4**

Weiterhin ist für die Haut die Durchlässigkeit für Wasserdampf von besonderer Bedeutung. Analoge Pflasterbinden mit ungeschäumten Heißschmelzklebemassen sind nicht wasserdampfdurchlässig. Die dargestellten Proben sind bei 23,5 °C vorkonditioniert worden. Als Prüfparameter sind beispielhaft die Temperatur 37 °C, der Sättigungsdampfdruck 6,274 kPa und die relative Luftfeuchtigkeit 30 % genannt.

Die anschließende Tabelle zeigt die Wasserdampfdurchlässigkeitsrate in Abhängigkeit vom Masseauftrag.

| Masseauftrag | Gewebe (unelast). in g/m$^2$ /24h | Gewebe (elast.) ungedehnt in g/m$^2$/24h | Vlies in g/m$^2$ /24h |
|---|---|---|---|
| 40 g/m$^2$ | 1020 | | |
| 60 g/m$^2$ | | | 2740 |
| 80 g/m$^2$ | 520 | 2510 | |
| 120 g/m$^2$ | 138 | | |

**Beispiel 5**

Im folgenden wird die Abhängigkeit der Wasserdampfdurchlässigkeit vom Schäumungsgrad gezeigt. Hierfür wurde ein Masseauftrag von 80 g/m$^2$ gewählt. Die unter Beispiel 4 beschriebenen Parameter sind konstant geblieben.

Die anschließende Tabelle zeigt die Wasserdampfdurchlässigkeitsrate in Abhängigkeit vom Schäumungsgrad.

| Schäumungsgrad | unelast. Gewebe in g/m$^2$ /24h |
|----------------|----------------------------------|
| 30% | 240 |
| 50% | 520 |
| 70% | 990 |

Muster mit der gleichen Klebemasse, welche jedoch ungeschäumt ist, weisen keine oder deutlich geringere Durchlässigkeiten auf.

Vorteilhaft insbesondere für die Verwendung bei medizinischen Produkten ist weiterhin, wenn die geschäumte Klebemasse partiell auf dem Trägermaterial aufgetragen ist, beispielsweise durch Rasterdruck, Thermosiebdruck, Thermoflexodruck oder Tiefdruck, denn im Vollstrich selbstklebend ausgerüstete Trägermaterialien können unter ungünstigen Voraussetzungen bei der Applikation mechanische Hautirritationen hervorrufen.

Der partielle Auftrag ermöglicht durch geregelte Kanäle die Abführung des transepidermalen Wasserverlustes und verbessert das Ausdampfen der Haut beim Schwitzen insbesondere bei der Verwendung von luft- und wasserdampfdurchlässigen Trägermaterialien. Hierdurch werden Hautirritationen, die durch Stauungen der Körperflüssigkeiten hervorgerufen werden, vermieden. Die angelegten Abführungskanäle ermöglichen ein Ableiten.

Die Klebemasse wird in Form von polygeometrischen Kalotten und ganz besonders von solchen Kalotten, bei denen das Verhältnis Durchmesser zu Höhe kleiner 5:1 ist aufgetragen.

Die geschäumte Klebemasse kann gleichmäßig auf dem Trägermaterial verteilt sein, sie kann aber auch funktionsgerecht für das Produkt über die Fläche unterschiedlich stark oder dicht aufgetragen sein. Schließlich kann die Kalotte auch auf eine Klebefläche zumindest teilweise aufgetragen werden.

Das Prinzip des Thermosiebdrucks besteht in der Verwendung einer rotierenden beheizten, nahtlosen, trommelförmigen perforierten Rundschablone, die über eine Düse mit der bevorzugten Heißschmelzklebemasse beschickt wird. Eine speziell geformte Düsenlippe (Rund- oder Vierkantrakel) presst die über einen Kanal eingespeiste geschäumte Heißschmelzklebemasse durch die Perforation der Schablonenwand auf die vorbeigeführte Trägerbahn. Diese wird mit einer Geschwindigkeit, die der Umgangsgeschwindigkeit der rotierenden Siebtrommel entspricht, mittels einer Gegendruckwalze gegen den Außenmantel der beheizten Siebtrommel geführt.

Die Ausbildung der kleinen Klebstoffkalotten geschieht dabei nach folgendem Mechanismus:

Der Düsenrakeldruck fördert die geschäumte Heißschmelzklebemasse durch die Siebperforation an das Trägermaterial. Die Größe der ausgebildeten Kalotten wird durch den Durchmesser des Siebloches vorgegeben. Entsprechend der Transportgeschwindigkeit der Trägerbahn (Rotationsgeschwindigkeit der Siebtrommel) wird das Sieb vom Träger abgehoben. Bedingt durch die hohe Adhäsion der Selbstklebemasse und die innere Kohäsion des Hotmelts wird von der auf dem Träger bereits haftenden Basis der Kalotten der in den Löchern begrenzte Vorrat an Heißschmelzklebemasse konturenscharf abgezogen beziehungsweise durch den Rakeldruck auf den Träger gefördert.

Nach Beendigung dieses Transportes formt sich, abhängig von der Rheologie der Heißschmelzklebemasse, über der vorgegebenen Basisfläche die mehr oder weniger stark gekrümmte Oberfläche der Kalotte. Das Verhältnis Höhe zur Basis der Kalotte hängt vom Verhältnis Lochdurchmesser zur Wandstärke der Siebtrommel und den physikalischen Eigenschaften (Fließverhalten, Oberflächenspannung und Benetzungswinkel auf dem Trägermaterial) der Selbstklebemasse ab.

Bei der Siebschablone im Thermosiebdruck kann das Steg/Loch-Verhältnis kleiner 3:1 sein, bevorzugt kleiner oder gleich 1:1, insbesondere gleich 1:3.

Der beschriebene Bildungsmechanismus der Kalotten erfordert bevorzugt saugfähige oder zumindest von Heißschmelzklebemasse benetzbare Trägermaterialien. Nichtbenetzende Trägeroberflächen müssen durch chemische oder physikalische Verfahren vorbehandelt werden. Dies kann durch zusätzliche Maßnahmen wie zum Beispiel Coronaentladung oder Beschichtung mit die Benetzung verbessernden Stoffen geschehen.

Mit dem aufgezeigten Druckverfahren kann die Größe und Form der Kalotten definiert festgelegt werden. Die für die Anwendung relevanten Klebkraftwerte, die die Qualität der erzeugten Produkte bestimmen, liegen bei sachgerechter Beschichtung in sehr engen Toleranzen. Der Basisdurchmesser der Kalotten kann von 10 μm bis 5000 μm gewählt werden, die Höhe der Kalotten von 20 μm bis 2000 μm, bevorzugt 50 μm bis 1000 μm, wobei der Bereich kleiner Durchmesser für glatte Träger, der mit größerem Durchmesser und größerer Kalottenhöhe für rauhe oder stark porige Trägermaterialien vorgesehen ist.

Die Positionierung der Kalotten auf dem Träger wird durch die in weiten Grenzen variierbare Geometrie des Auftragswerkes, zum Beispiel Gravur- oder Siebgeometrie, definiert festgelegt. Mit Hilfe der aufgezeigten Parameter kann über einstellbare Größen das gewünschte Eigenschaftsprofil der Beschichtung, abgestimmt auf die verschiedenen Trägermaterialien und Anwendungen, sehr genau eingestellt werden.

Das Trägermaterial wird bevorzugt mit einer Geschwindigkeit von größer 2 m/min, bevorzugt 20 bis 220 m/min, beschichtet, wobei die Beschichtungstemperatur größer als die Erweichungstemperatur zu wählen ist.

Die geschäumte Heißschmelzklebemasse kann mit einem Flächengewicht von größer 15 g/m$^2$, bevorzugt zwischen 30 g/m$^2$ und 400 g/m$^2$, ganz besonders bevorzugt zwischen 130 g/m$^2$ und 300 g/m$^2$, auf dem Trägermaterial aufgetragen sein.

Der prozentuale Anteil, der mit der geschäumten Heißschmelzklebemasse beschichteten Fläche sollte mindestens 20 % betragen und kann bis zu ungefähr 95 % reichen, für spezielle Produkte bevorzugt 40 % bis 60 % sowie 70 % bis 95 %. Dieses kann gegebenenfalls durch Mehrfachapplikation erreicht werden, wobei gegebenenfalls auch geschäumte Heißschmelzklebemassen mit unterschiedlichen Eigenschaften eingesetzt werden können.

Die Kombination der geschäumten Heißschmelzklebemasse und der partiellen Beschichtung sichert auf der einen Seite eine sichere Verklebung des medizinischen Produktes auf der Haut, auf der anderen Seite sind zumindest visuell erkennbare allergische oder mechanische Hautirritationen ausgeschlossen, auch bei einer Anwendung, die sich über mehrere Tage erstreckt.

Die Epilation entsprechender Körperregionen und der Massetransfer auf die Haut sind aufgrund der hohen Kohäsivität des Klebers vernachlässigbar, weil der Kleber nicht an Haut und Haar verankert, vielmehr ist die Verankerung der Klebemasse auf dem Trägermaterial mit bis zu 15 N/cm (Probenbreite) für medizinische Anwendungen gut.

Durch die ausgeformten Sollbruchstellen in der Beschichtung werden Hautlagen beim Ablösen nicht mehr mit- oder gegeneinander verschoben. Das Nichtverschieben der Hautlagen und die geringere Epilation führen zu einem bisher nicht gekannten Grad der Schmerzfreiheit bei solchen stark klebenden Systemen. Weiter unterstützt die individuelle biomechanische Klebkraftsteuerung, welche eine nachweisliche Absenkung der Klebkraft dieser Pflaster aufweist, die Ablösbarkeit. Das applizierte Pflaster zeigt gute propriorezeptive Wirkungen.

Je nach Trägermaterial und dessen Temperaturempfindlichkeit kann die geschäumte Heißschmelzklebemasse direkt aufgetragen sein oder zuerst auf einen Hilfsträger aufgebracht und dann auf den endgültigen Träger transferiert werden. Auch ein nachträgliches Kalandern des beschichteten Produktes und/oder eine Vorbehandlung des Trägers, wie Coronabestrahlung, zur besseren Verankerung der Klebeschicht kann vorteilhaft sein.

Weiterhin kann eine Behandlung der geschäumten Heißschmelzklebemasse mit einer Elektronenstrahl-Nachvemetzung oder einer UV-Bestrahlung zu einer Verbesserung der gewünschten Eigenschaften führen.

Als Trägermaterialien eignen sich alle starren und elastischen Flächengebilde aus synthetischen und natürlichen Rohstoffen. Bevorzugt sind Trägermaterialien die nach Applikation der Klebemasse so eingesetzt werden können, dass sie Eigenschaften eines funktionsgerechten Verbandes erfüllen.

Beispielhaft sind Textilien wie Gewebe, Gewirke, Gelege, Vliese, Laminate, Netze, Folien, Schäume und Papiere aufgeführt. Weiter können diese Materialien vor- beziehungsweise nachbehandelt werden. Gängige Vorbehandlungen sind Corona und Hydrophobieren; geläufige Nachbehandlungen sind Kalandern, Tempern, Kaschieren, Stanzen und Eindecken.

Das mit der geschäumten Klebemasse beschichtete Trägermaterial hat eine Luftdurchlässigkeit von größer 1 cm$^3$/(cm$^2$*s), bevorzugt größer 15 cm$^3$/(cm$^2$*s), ganz besonders bevorzugt größer 50 cm$^3$/(cm$^2$*s), des weiteren eine Wasserdampfdurchlässigkeit von größer 500 g/(m$^2$*24h), bevorzugt größer 1000 g/(m$^2$*24h), ganz besonders bevorzugt größer 2000 g/(m$^2$*24h).

Die Luft- und Wasserdampfdurchlässigkeit ist auch bei einem mehrlagigen Verkleben gegeben.

Schließlich kann das Pflaster nach dem Beschichtungsvorgang mit einem klebstoffabweisenden Trägermaterial, wie silikonisiertes Papier, eingedeckt oder mit einer Wundauflage oder einer Polsterung versehen werden. Anschließend werden die Pflaster in der gewünschten Größe ausgestanzt.

Besonders vorteilhaft ist, dass das Pflaster sterilisierbar, bevorzugt γ-(gamma) sterilisierbar, ist. So sind besonders geeignet für eine nachträgliche Sterilisation Heißschmelzklebemassen auf Blockcopolymerbasis, welche keine Doppelbindungen enthalten. Dieses gilt ins besondere für Styrol-Butylen-Ethylen-Styrol-Blockcopolymerisate oder Styrol-Butylen-Styrol-Blockcopolymerisate. Es treten hierbei keine für die Anwendung signifikanten Änderungen in den Klebeeigenschaften auf.

Das erfindungsgemäße Pflaster weist eine Klebkraft auf der Trägerrückseite von mindestens 1,5 N/cm auf, besonders eine Klebkraft zwischen 2,5 N/cm und 5 N/cm. Auf anderen Untergründen können höhere Klebkräfte erreicht werden.

Im folgenden sollen besonders vorteilhafte Pflaster der Erfindung beschreiben werden, ohne die Erfindung dadurch unnötig einschränken zu wollen.

**Beispiel 6**

Erfindungsgemäß wurde eine Vorrichtung zur Freigabe von Stoffen hergestellt, welche einen hyperämisieren Wirkstoff enthält. Die Vorrichtung kann aufgrund ihrer nachfolgend beschriebenen Eigenschaften zur Anwendung als Rheumapflaster dienen, welches auch aufgrund der guten klebetechnischen Eigenschaften mehrtägig an Gelenken des menschlichen Bewegungsapparates zu applizieren ist.

Das Trägermaterial bestand aus einem unelastischen Baumwollgewebe mit einer Höchstzugkraft von größer 80 N/cm und einer Höchstzugkraft-Dehnung von kleiner 30%.

Diese Schmelzhaftklebemasse setzte sich wie folgt zusammen:

- ein A-B/A-B-A Blockcopolymer, welches aus harten und weichen Segmenten besteht, mit einem Verhältnis der A-B-A zur A-B von 2:1 und einem Styrolgehalt im Polymer von 13 Mol.-%; der Anteil an der Klebemasse beträgt 44 Gew.-% (Kraton G)
- ein Paraffinkohlenwasserstoffwachs mit einem Anteil an der Klebmasse von 52 Gew.-%
- Kohlenwasserstoffharze mit einem Anteil von 3,5 Gew.-% (Super Resin HC 140)
- ein Alterungsschutzmittel mit einem Anteil von weniger als 0,5 Gew.-% (Irganox 1010)
- ein hyperämisierender Wirkstoff (Nonanonyl Vanillylamid) mit einem Anteil von 1 Gew.-%

Die eingesetzten Kleberkomponenten wurden in einem Thermomischer bei 185°C drei Stunden homogenisiert. Der Wirkstoff wurde in der Abkühlphase bei 130 °C zugegeben und weitere 30 min im Mischer homogenisiert. Der Erweichungspunkt dieser Klebmasse betrug 85 °C (DIN 52011), und die Klebmasse zeigte eine Viskosität von 2100 mPas bei 150 °C (DIN 53018, Brookfield DV II, Sp. 21). Die Glasübergang betrug nach oben genannter Methode -9 °C.

Die so hergestellte dotierte Heißschmelzklebemasse wurde in einem Foam-Mix-Gerät der Firma Nordson aufgeschäumt. Als Schaumgas wurde Stickstoff verwendet. Der Schäumungsgrad betrug 70 % vor der Auftragsdüse.

Die Selbstklebemasse wurde mit einer Düse vollflächig auf den Träger appliziert. Die direkte Beschichtung erfolgte mit 50 m/min bei einer Temperatur von 120 °C. Das Trägermaterial wurde mit 170 g/m$^2$ beschichtet.

Das so hergestellte Pflastermaterial zeigt eine vergleichbar gute Freisetzung (Uberationsstudie) des Wirkstoffs. Nach 24-stündiger in vitro-Anwendung an Schweinehaut wurden 15% der Pflasterladung dermal absorbiert.

Die Luftdurchlässigkeit des ausgerüsteten Trägermaterials betrug 15 cm$^3$/(cm$^2$*s). Nach der Applikation wurden keine Hautirritationen festgestellt.

## Patentansprüche

1. Wirkstoffhaltige Pflaster mit einem Trägermaterial und einer darauf partiell aufgebrachten Heißschmelzklebemasse, **dadurch gekennzeichnet, dass** die Heißschmelzklebemasse

   a) 10 Gew.% bis 90 Gew.% A-B/A-B-A- Blockcopolymeren mit einem Anteil an Di-Blockcopolymeren von weniger als 80 Gew.%,

   b) wobei Phase A vornehmlich Polystyrol oder dessen Derivate sind und der Gesamtstyrolanteil in der Klebemasse weniger als 35 Gew.% beträgt,

   c) 5 Gew.% bis 80 Gew.% Klebrigmacher, insbesondere Öle, Wachse, Harze und/oder deren Mischungen,

   d) weniger als 60 Gew.% Weichmacher,

   e) weniger als 15 Gew.% Additive,

   f) weniger als 5 Gew.% Stabilisatoren und

   g) 0,01 Gew.% bis 10 Gew.% Wirkstoff oder Wirkstoffe enthält und

   h) eine dynamisch-komplexe Glasübergangstemperatur bei einer Frequenz von 0,1 rad/s von weniger als 10 °C aufweist,

   i) die offenporige Klebemasse mittels dem Schaum-Mix-System mit einem Schäumungsgrad von mindestens 5 Vol.-% geschäumt ist und

   j) in Form von polygeometrischen Kalotten auf das Trägermaterial aufgebracht ist und

   k) dass das mit Klebemasse beschichtete Trägermaterial eine Luftdurchlässigkeit größer 1 cm$^3$/cm$^2$ * s) und eine Wasserdampfdurchlässigkeit größer 500 g/(m$^2$*24h) aufweist.

2. Wirkstoffhaltige Pflaster gemäß Anspruch 1, **dadurch gekennzeichnet, dass** die Phase B Ethylen, Propylen, Butylen, Butadien, Isopren oder deren Mischungen, hierbei besonders bevorzugt Ethylen und Butylen oder deren Mischungen, sind.

3. Wirkstoffhaltige Pflaster gemäß Anspruch 1 oder 2, **dadurch gekennzeichnet, dass** der gesamte Styrolanteil im Polymer zwischen 5 Gew.% bis 30 Gew.% liegt.

4. Wirkstoffhaltige Pflaster gemäß mindestens einem der vorhergehenden Ansprüche, **dadurch gekennzeichnet, dass** die Heißschmelzklebemasse bei einer Frequenz von 0,1 rad/s eine dynamisch-komplexe Glasübergangstemperatur von 0 °C bis -30 °C, besonders bevorzugt von -6 °C bis -25 °C, aufweist.

5. Wirkstoffhaltige Pflaster gemäß mindestens einem der vorhergehenden Ansprüche, **dadurch gekennzeichnet, dass** die Klebemasse mit einem Flächengewicht von größer 15 g/m$^2$, bevorzugt zwischen 30 g/m$^2$ und 400 g/m$^2$, ganz besonders bevorzugt zwischen 130 g/m$^2$ und 300 g/m$^2$, auf dem Trägermaterial aufgetragen ist.

6. Wirkstoffhaltige Pflaster gemäß mindestens einem der vorhergehenden Ansprüche, **dadurch gekennzeichnet, dass** der prozentuale Anteil der mit der Klebemasse beschichteten Fläche mindestens 20 % beträgt, bevorzugt 40 % bis 60 %, ganz besonders bevorzugt 70 % bis 95 %.

7. Wirkstoffhaltige Pflaster gemäß mindestens einem der vorhergehenden Ansprüche, **dadurch gekennzeichnet, dass** das mit der geschäumten Klebemasse beschichtete Trägermaterial eine Luftdurchlässigkeit von größer 15 cm$^3$/(cm$^2$*s), ganz besonders bevorzugt größer 50 cm$^3$/(cm$^2$*s), und eine Wasserdampfdurchlässigkeit von größer 1000 g/(m$^2$*24h), ganz besonders bevorzugt größer 2000 g/(m$^2$*24h) aufweist.

8. Wirkstoffhaltige Pflaster gemäß mindestens einem der vorhergehenden Ansprüche, **dadurch gekennzeichnet, dass** das Pflaster sterilisiert, bevorzugt γ (gamma)-sterilisiert, ist.

9. Wirkstoffhaltige Pflaster gemäß mindestens einem der vorhergehenden Ansprüche, **dadurch gekennzeichnet, dass** das Pflaster eine Klebkraft auf der Trägerrückseite von mindestens 1,5 N/cm, besonders eine Klebkraft zwischen 2,5 N/cm und 5 N/cm, aufweist.

10. Verfahren zur Herstellung eines wirkstoffhaltigen Pflasters nach einem der vorstehenden Ansprüche, **dadurch gekennzeichnet, dass** die Klebemassenbestandteile gezielt abgemischt werden, die dynamisch-komplexe Glasübergangstemperatur der Klebemasse bei einer Frequenz von 0,1 rad/s auf weniger als 10 °C abgesenkt wird und nach dem Schaum-Mix-System mit den vorgesehen Gasen geschäumt und in Form von polygeometrischen Kalotten auf das Trägermaterial aufgebracht werden.

11. Verwendung eines wirkstoffhaltigen Pflasters nach einem der vorstehenden Ansprüche 1 bis 9 zur Herstellung eines Arzneimittels zur Verwendung als Rheumapflaster.

**Claims**

1. Active substance plasters comprising a backing material which is at least partially coated with a hotmelt adhesive composition, **characterized in that** the hotmelt adhesive composition comprises

   a) from 10 to 90% by weight of A-B/A-B-A block copolymers having a proportion of diblock copolymers of less than 80% by weight,
   b) phase A being primarily polystyrene or derivatives thereof and the overall proportion of styrene in the adhesive composition being less than 35% by weight,
   c) from 5 to 80% by weight of tackifiers, especially oils, waxes, resins and/or mixtures thereof,
   d) less than 60% by weight of plasticizers,
   e) less than 15% by weight of additives,
   f) less than 5% by weight of stabilizers, and
   g) from 0.01 to 10% by weight of active substance or substances, and
   h) has a dynamic-complex glass transition temperature of less than 10°C at a frequency of 0.1 rad/s,
   i) the open-pored adhesive composition has been foamed by means of the foam mixing system with a degree of foaming of at least 5% by volume and
   j) has been applied in the form of polygeometric domes to the backing material, and
   k) **in that** the adhesive-coated backing material has a permeability to air of greater than 1 cm$^3$/(cm$^2$ * s) and a permeability to water vapour of greater than 500 g/(m$^2$*24 h).

2. Active substance plasters according to Claim 1, **characterized in that** phase B comprises ethylene, propylene, butylene, butadiene, isoprene or mixtures thereof, with particular preference ethylene and butylene or mixtures thereof.

3. Active substance plasters according to Claim 1 or 2, **characterized in that** the overall proportion of styrene in the polymer is between 5 to 30% by weight.

4. Active substance plasters according to at least one of the preceding claims, **characterized in that** the hot melt adhesive composition has a dynamic-complex glass transition temperature of 0°C to -30°C, more preferably of -6°C to -25°C, at a frequency of 0.1 rad/s.

5. Active substance plasters according to at least one of the preceding claims, **characterized in that** the adhesive composition has been applied to the backing material with an areal weight of greater than 15 g/m$^2$, preferably between 30 g/m$^2$ and 400 g/m$^2$, very preferably between 130 g/m$^2$ and 300 g/m$^2$.

6. Active substance plasters according to at least one of the preceding claims, **characterized in that** the percentage fraction of the area coated with the adhesive composition is at least 20%, preferably 40% to 60%, very preferably 70% to 95%.

7. Active substance plasters according to at least one of the preceding claims, **characterized in that** the backing material coated with the foamed adhesive composition has a permeability to air of greater than 15 cm$^3$/(cm$^2$*s), very preferably greater than 50 cm$^3$/(cm$^2$*s), and a permeability to water vapour of greater than 1000 g/(m$^2$*24 h), very preferably greater than 2000 g/(m$^2$*24 h).

8. Active substance plasters according to at least one of the preceding claims, **characterized in that** the plaster has been sterilized, preferably by means of $\gamma$ (gamma) radiation.

9. Active substance plasters according to at least one of the preceding claims, **characterized in that** the plaster has a bond strength on the reverse of the backing of at least 1.5 N/cm, in particular a bond strength of between 2.5 N/cm and 5 N/cm.

10. Process for preparing an active substance plaster according to any one of the preceding claims, **characterized in that** the constituents of the adhesive composition are purposively blended, the dynamic-complex glass transition temperature of the adhesive composition is lowered to less than 10°C at a frequency of 0.1 rad/s and it is foamed by the foam mixing system with the intended gases and is applied in the form of polygeometric domes to the backing material.

11. Use of an active substance plaster according to any one of Claims 1 to 9 above for producing a medicinal product for use as a rheumatism patch.

## Revendications

1. Pansement contenant une ou plusieurs substances actives comprenant un matériau support et une masse adhésive thermofusible appliquée partiellement sur celui-ci, **caractérisé en ce que** la masse adhésive thermofusible

   a) contient 10% en poids à 90% en poids de copolymères à blocs A-B/A-B-A présentant une proportion de copolymères à deux blocs inférieure à 80% en poids,
   b) la phase A étant essentiellement du polystyrène ou ses dérivés et la proportion totale de styrène dans la masse adhésive étant inférieure à 35% en poids,
   c) 5% en poids à 80% en poids d'agents poisseux, en particulier les huiles, les cires, les résines et/ou leurs mélanges,
   d) moins de 60% en poids de plastifiants,
   e) moins de 15% en poids d'additifs,
   f) moins de 5% en poids de stabilisateurs et
   g) 0,01% en poids à 10% en poids d'une ou de plusieurs substances actives et
   h) présente une température de transition vitreuse dynamique-complexe à une fréquence de 0,1 rad/s inférieure à 10°C,
   i) la masse adhésive à pores ouverts est moussée au moyen du système de moussage-mélange à un degré de moussage d'au moins 5% en poids et
   j) est appliquée sous forme de calottes polygéométriques sur le matériau support et
   k) le matériau support revêtu par la masse adhésive présente une perméabilité à l'air supérieure à 1 cm$^3$/(cm$^2$*s) et une perméabilité à la vapeur d'eau supérieure à 500 g/(m$^2$*24h).

2. Pansement contenant une ou plusieurs substances actives selon la revendication 1, **caractérisé en ce que** la phase B est constituée par l'éthylène, le propylène, le butylène, le butadiène, l'isoprène ou leurs mélanges, en préférant en particulier l'éthylène et le butylène ou leurs mélanges.

3. Pansement contenant une ou plusieurs substances actives selon la revendication 1 ou 2, **caractérisé en ce que** la proportion totale de styrène dans le polymère est comprise entre 5% en poids et 30% en poids.

4. Pansement contenant une ou plusieurs substances actives selon au moins l'une quelconque des revendications précédentes, **caractérisé en ce que** la masse adhésive thermofusible présente, à une fréquence de 0,1 rad/s une température de transition vitreuse dynamique-complexe de 0°C à -30°C, de manière particulièrement préférée de -6°C à -25°C.

5. Pansement contenant une ou plusieurs substances actives selon au moins l'une quelconque des revendications précédentes, **caractérisé en ce que** la masse adhésive est appliquée sur le matériau support à un poids surfacique supérieur à 15 g/m$^2$, de préférence entre 30 g/m$^2$ et 400 g/m$^2$, de manière tout particulièrement préférée entre 130 g/m$^2$ et 300 g/m$^2$.

6. Pansement contenant une ou plusieurs substances actives selon au moins l'une quelconque des revendications précédentes, **caractérisé en ce que** la proportion en pour-cent de la surface revêtue par la masse adhésive représente au moins 20%, de préférence 40% à 60%, de manière tout particulièrement préférée 70% à 95%.

7. Pansement contenant une ou plusieurs substances actives selon au moins l'une quelconque des revendications précédentes, **caractérisé en ce que** le matériau support revêtu par la masse adhésive moussée présente une perméabilité à l'air supérieure à 15 cm$^3$/(cm$^2$*s), de manière tout particulièrement préférée supérieure à 50 cm$^3$/(cm$^2$*s), et une perméabilité à la valeur d'eau supérieure à 1000 g/(m$^2$*24h), de manière tout particulièrement préférée supérieure à 2000 g/(m$^2$*24h).

8. Pansement contenant une ou plusieurs substances actives selon au moins l'une quelconque des revendications précédentes, **caractérisé en ce que** le pansement est stérilisé, de préférence stérilisé par des rayons γ (gamma).

9. Pansement contenant une ou plusieurs substances actives selon au moins l'une quelconque des revendications précédentes, **caractérisé en ce que** le pansement présente une adhésivité sur le dos du support d'au moins 1,5 N/cm, en particulier une adhésivité entre 2,5 N/cm et 5 N/cm.

10. Procédé pour la réalisation d'un pansement contenant une ou plusieurs substances actives selon l'une quelconque des revendications précédentes, **caractérisé en ce que** les constituants de la masse adhésive sont mélangés de manière ciblée, la température de transition vitreuse dynamique-complexe de la masse adhésive est abaissée, à une fréquence de 0,1 rad/s, à moins de 10°C et ils sont moussés selon le système de moussage-mélange avec les gaz prévus et appliqués sous forme de calottes polygéométriques sur le matériau support.

11. Utilisation d'un pansement contenant une ou plusieurs substances actives selon l'une quelconque des revendications précédentes 1 à 9 pour la réalisation d'un médicament destiné à une utilisation comme pansement contre les rhumatismes.